# EUROPEAN PATENT SPECIFICATION

(11) **EP 1 168 967 B1**
(45) Date of publication and mention of the grant of the patent: **06.04.2005**
(21) Application number: 00920861.2
(22) Date of filing: 07.04.2000
(51) Int. Cl.: A61B 17/04

(54) **APPARATUS FOR SUTURING TISSUE**
VORRICHTUNG ZUM NÄHEM VON GEWEBE
DISPOSITIF DE SUTURE DE TISSU

(30) Priority: 09.04.1999 GB 9908098
(43) Date of publication of application: 09.01.2002
(73) Proprietor: Surgi-Call Limited, Sutton Coldfield, Birmingham B73 6JX (GB)
(72) Inventor: Kabukoba, Josaphat Joseph, Birmingham B73 6JX (GB)
(74) Representative: Evens, Paul Jonathan
(86) International application number: PCT/GB2000/001250
(87) International publication number: WO 2000/061012

(56) References cited:
- EP-A- 0 669 103
- WO-A-94/15535
- WO-A-96/22735
- DE-A- 4 317 328
- US-A- 4 932 962
- US-A- 5 725 542

## Description

### BACKGROUND OF THE INVENTION

### FIELD OF THE INVENTION

The present invention relates to apparatus for suturing.

### DESCRIPTION OF THE RELATED ART

Current suturing techniques, especially laparoscopic (keyhole) work is difficult and time consuming, involving many movements and instruments in generally confined spaces.

Surgical crimping devices and methods of use are described in European patent applications published under EP 0 669 101A and EP 0 669 103A. As shown in Figure 10, an applicator (110) is provided which is able to deform and compress in situ a securing member (120) around two ends (130,140) of suture material (200) which is received therethrough and pulled taut. The securing member (120) is able to maintain a prescribed amount of tension on the suture which extends through tissue (not shown), even after the applicator (110) is removed.

Other techniques have evolved including stapling. Such techniques and systems are either cumbersome or expensive and are not applicable in many situations. Ligatures in the forms of clips have also been provided but these can only be used with tissue that is relatively small - for example, a blood vessel. Clips on their own are not very secure.

An apparatus with the features of the first part of claim 1 is known from WO-A-96/22735.

There is now proposed apparatus for suturing having, it is believed, many advantages over the known arrangements and techniques. The invention is as defined in the appended set of claims.

### SUMMARY OF THE INVENTION

In a first aspect, the present invention provides apparatus for suturing as defined in claim 1.

With a loop of suture material, tissue may be sutured by threading the leading part through an exposed part of the loop, perhaps adjacent the trailing part. Thereafter, pulling the leading part away from the remainder of the loop has the effect of drawing the noose-like suture tight. It is then necessary to secure the suture against unravelling. With the present invention, the chosen suture tension is maintained by the suture clip. In use, the suture clip engages two sections of the loop of suture material and bears against the trailing part of the loop or surrounding tissue. The suture clip effectively locks the two sections of the loop relative to the trailing part of the loop.

The body of the suture clip may have a tubular portion. In which case, the leading part of the loop would be threaded through the opening or bore of the tubular portion to bring the suture clip into its operable position. Alternatively, the body of the suture clip may have a grooved portion (i.e. U-shaped section) with an open recess into which the two sections of the loop may be slotted. In either case, the engaging means may be provided by a pair of opposed surfaces of the body which are movable between an inoperative position where suture loop sections are not engaged and an operative position where suture loop sections are engaged - i.e. trapped between the opposed pair of surfaces.

The pair of opposed surfaces may move from the inoperative position to the operative position through plastic deformation within the body. The body with a tubular portion may be crushable, and a body with a grooved portion (i.e. U-shaped section) may be foldable. Alternatively, the pair of opposes surfaces may move between the inoperative and operative positions through elastic deformation or hinged movement within the body, in which case a locking member is provided to maintain the opposed surfaces in the operative position when required. The pair of opposed surfaces may include adhesive to either adhere direct to the suture loop sections or bond the opposed surfaces together when brought into the operative position.

The peripheral surfaces of the suture clip may be bonded to the loop of suture material, either before or after the suture has been formed. For example, the suture clip may be welded to the trailing part of the suture material. Alternatively, the peripheral surface of the suture clip may simply abut the trailing part or surrounding tissue, thereby acting as a block against the suture unravelling. In the latter case, the peripheral surface of the suture clip may have a profile for preventing the trailing part of the loop from slipping past or riding over the suture clip. The profile essentially provides an enlarged suture clip footprint in a plane substantially perpendicular to the orientation of suture loop sections when retained in the suture clip. The profile may be provided by a flared member which, in use, surrounds a region of contact between the leading and trailing parts of the loop and may even bear against adjacent tissue.

The loop of suture material may be provided by joining the two free ends of a length of suture material. The two free ends may be knotted together or clipped together. In the latter case, the join may be achieved with a suture slip as hereinbefore defined which is bonded to the resulting loop. The leading part of the loop of suture material may be coupled to a surgical needle.

The apparatus may further comprise an applicator for the suture clip, the applicator having means for applying tension to the loop of suture material during suturing, and means for applying the suture clip to secure the suture. The applicator may further comprise means for cutting excess suture material from the suture when secured.

It will be appreciated that a looped suture brought onto itself over tissue and under tension will be in direct and firm contact at the point of engagement as long as the tension in the suture material is maintained.

It will be appreciated that the apparatus of the invention may be used in both open and laparoscopic surgery.

### BRIEF DESCRIPTION OF THE DRAWINGS

The above and other aspects, features and advantages will become apparent from the following description of embodiments of the invention now made with reference to the accompanying drawings, in which:-
Figures 1 to 4 illustrate a suturing technique embodying the present invention;
Figure 5 shows at A and B respectively a first suture clip embodying the invention immediately before and after application to the suture material;
Figure 6 shows at A and B respectively, side views of another embodiment of a suture slip in accordance with the invention;
Figures 7 and 8 A-C illustrate other forms of the suture clip embodying the invention;
Figure 9 is a flow chart illustrating a suturing method in accordance with the invention; and

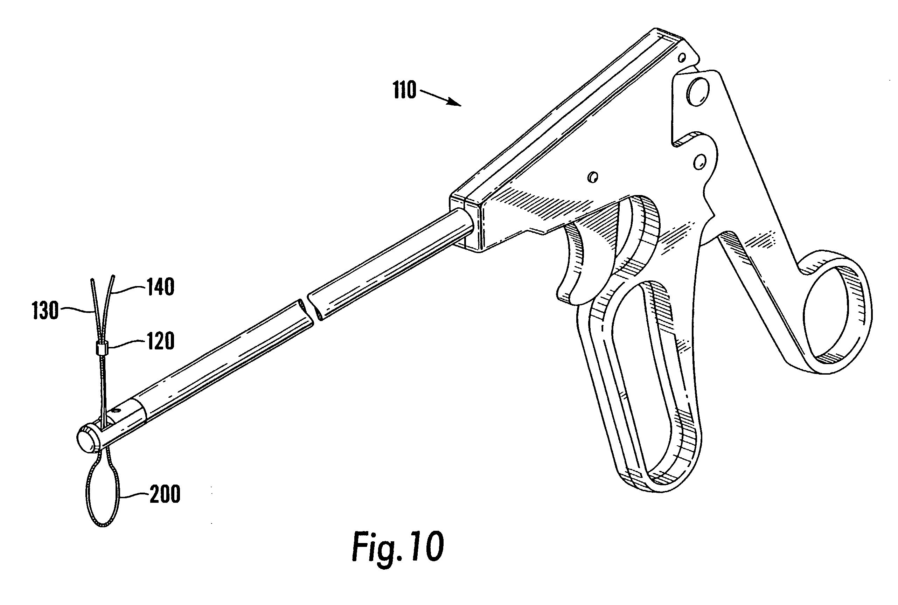

Figure 10 shows a prior art arrangement for the sake of comparison.

### DESCRIPTION OF EMBODIMENTS OF THE INVENTION

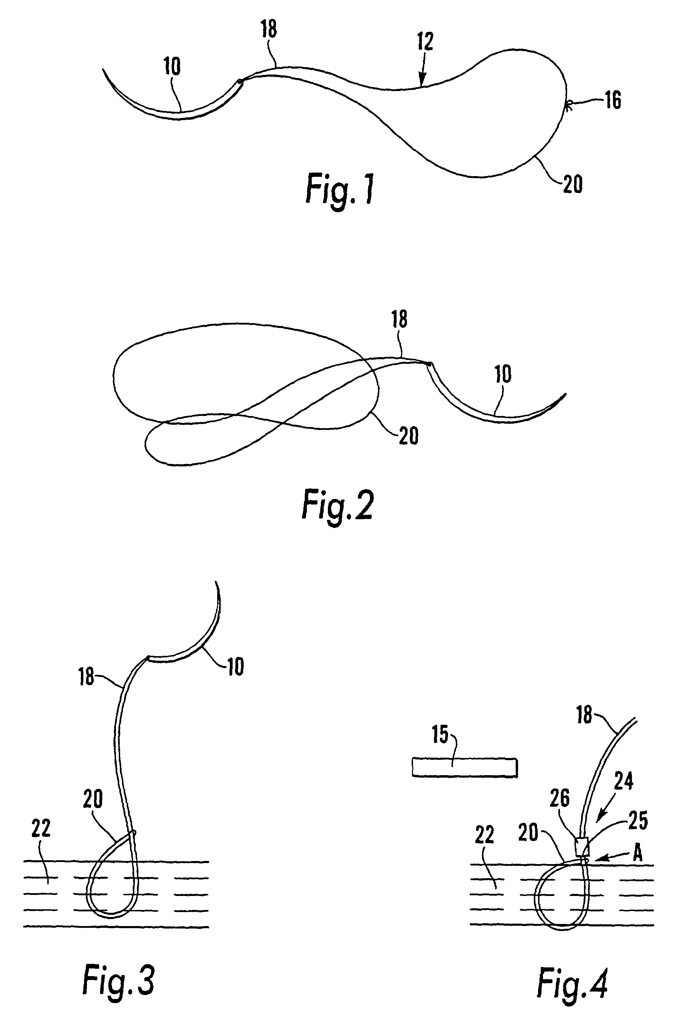

Referring firstly to Figures 1 to 4 which show a steel needle (10) which is curved (although it may be straight) through the eye of which is passed a loop (12) of Nylon suture material. (The suture material is illustrated with a single line and hence without thickness, for the sake of clarity). The loop (12) may be formed by knotting at (16) and has a leading part (18) adjacent the needle and a trailing part (20) remote or spaced from the needle. The needle (10) is passed through tissue (22) being sutured and thereafter through the loop (12) pulling the leading part (18) through and beyond the trailing part (20) of the loop (12).

In time the suture will be made with the suture material held under tension. Thereafter a suture clip (24) is positioned on the suture material adjacent the point (A) of engagement of the leading and trailing parts (18,20). The suture clip (24) has a body (26) which engages two side-by-side sections of the loop of suture material, thereby preventing movement of one section relative to the other. The body (26) has a peripheral surface (25) which engages or abuts either the trailing part (20) of the loop (12) or surrounding tissue. The suture clip (24) maintains tension in the suture by preventing the trailing part (20) from riding over or slipping past the point A. The suture clip (24) is positioned and tension applied to the suture using applicator (15). Excess suture material beyond the suture clip (24) may be cut off and discarded or over reused.

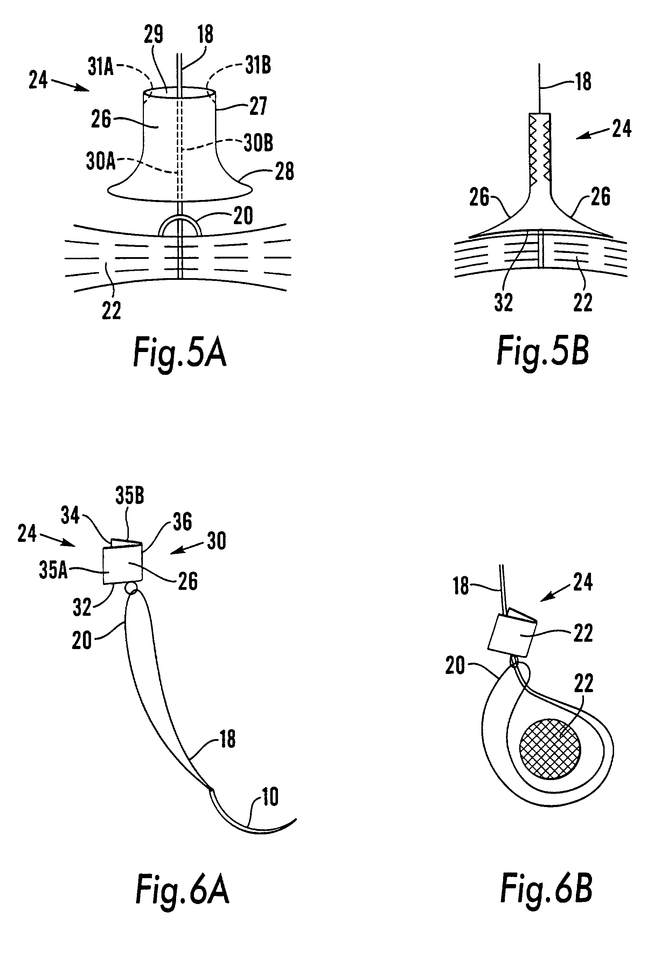

Various embodiments of the suture clip are illustrated in Figures 5-8. Wherever possible, the same reference numbers will be used for features in common.

Figure 5A and 5B show a suture clip (24) comprising a body (26) having a tubular portion (27) and a flared portion (28) with a bore (29) extending through the body (26). Once the suture has been formed (as per Figures 1-3), the suture clip (24) is brought into position by threading the leading part (18) through the bore (29) until the tubular portion (27) surrounds two sections (30A and 30B) of the loop of suture material. The suture clip (24) must then be secured in position in order to prevent the suture from unravelling.

The tubular part (27) of the suture clip (24) is crushable and it is this which provides the means for engaging the two sections (30A,30B). Initially, in the inoperative position, a pair of opposed surfaces (31A,31B) on the inner periphery of the bore (29) are sufficiently far apart to accommodate readily the two sections (30A, 30B). However, crushing the tubular part (27) brings the opposed surfaces (31A,31B) into the operative position where they trap the two sections (30A, 30B) between them. The flared portion (28) provides the peripheral surface (32) which bears against the trailing part (20). Even after crushing the tubular part (27), the peripheral surface (32) is significantly wider than the rest of the suture clip (24), and thus provides a stop, preventing the trailing part (20) from riding over the clip.

Figure 6A and 6B show a suture clip (24) comprising a body (26) which is attached to the midpoint of the trailing part (20) and is thus carried on the loop (12) before the suture is even formed. The body (26) has a grooved portion provided by a pair of wing-like members (35A,35B) which are mutually inclined to a fold line (36). The grooved portion has an open recess into which the two sections (30A,30B) of the loop (12) may be slotted. The facing surfaces of the wing-like members (35A,35B) provide the opposed surfaces (31A,31B). These surfaces may be brought into engagement with the two sections (30A,30B) by pushing the members (35A,35B) together and inducing plastic deformation around the fold line (36). The lower edges of the wing-like members (35A,35B) provide the peripheral surface (32) which in use bears against the trailing part (20).

It will be appreciated that the facing surfaces of the wing-like members (35A,35B) may be coated with adhesive. In this way, as soon as the suture material is pulled tight and the opposed surfaces (31A,31B) brought together, the wing-like members (35A, 35B) will grip the sections (30A, 30B) and hold them in place. The adhesive acts as a locking mechanism for maintaining the opposed surfaces in the operative position, even in the absence of plastic deformation around the fold line (36).

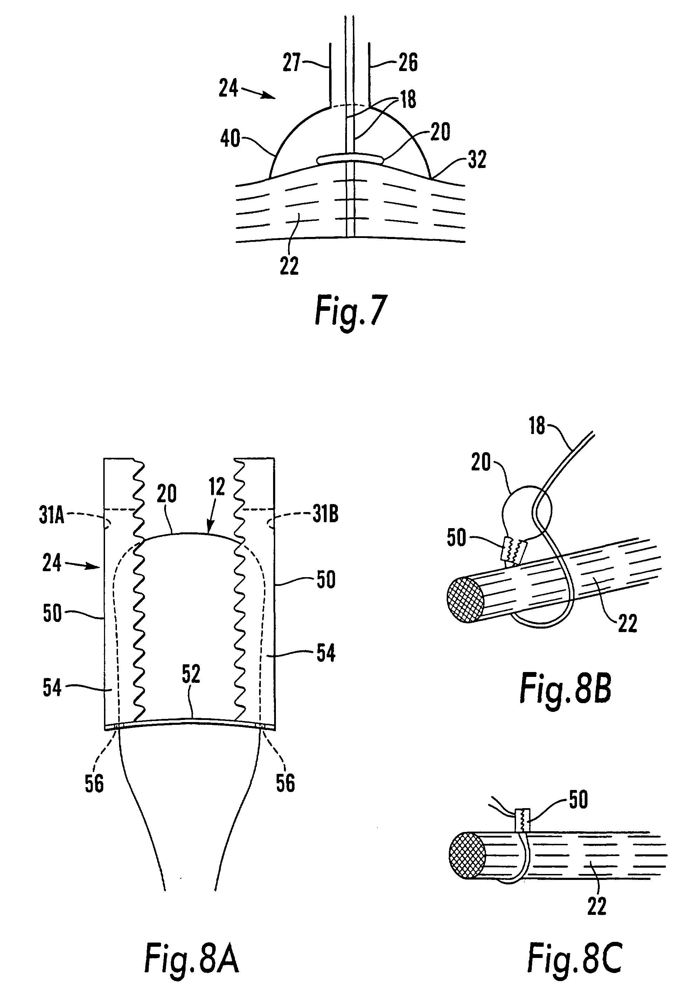

Figure 7 illustrates another form of suture clip (24) usable in place of the suture clip shown in Figure 5. The body (26) of the suture clip (24) shown in Figure 7 has a tubular portion (27) and a lower, as viewed in the Figure, bell shaped portion (40). When the clip (24) is correctly positioned over the point of engagement of the leading and trailing parts (18, 20) of the loop (12) of suture material and the tubular portion (27) is crushed so as to hold the suture material, the lower edge (i.e. surface 32) of portion (40) bears on the tissue (22) which has been sutured surrounding the point of engagement. In this way it will be appreciated that the suture clip (24) shown in Figure 7 cannot become released by the suture material riding over the clip once there is tension in the suture.

Figures 8A,B,C illustrate yet another form of suture clip (24) embodying the invention which is threaded onto the suture material before the loop is completed. The suture clip (24) has a pair of arms (50) first ends of which are joined to the ends of a flat plate like portion (52). The arms (50) have channels (54) through which the loop (12) of suture material is run after passing through apertures (56) in the plate like portion (52). It will be appreciated that the suture clip of Figure 8 is free to move along the suture material as a suture is being formed until the plate like portion (52) bears on the sutured tissue. After the suture has been formed and tension applied, the arms (50) are brought together and opposed surfaces (31A,31B) hold two sections (30A,30B) of the suture material to prevent the suture unravelling. It is to be noted that parts of the arms (50) extend beyond the point of engagement of leading and trailing parts of the loop (12) of suture material. The plate like portion (52) of the clip of Figure 8 is flexible to allow the arms (50) to be brought together and in use bears on the sutured tissue without damaging or traumatising the tissue.

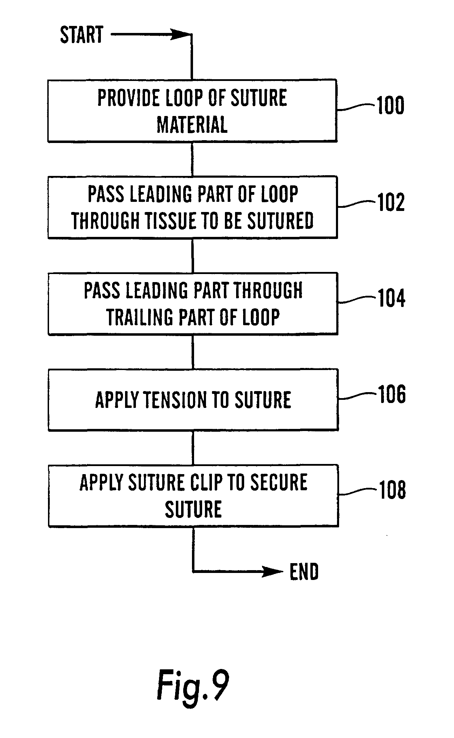

Figure 9 is a simple flow chart which corresponds to the steps identified in Figures 1 - 4. In other words, a method of suturing employing the apparatus of the present invention requires:
i) providing a loop of suture material (100);
ii) passing the leading part of the loop through tissue to be suture (102);
iii) passing the leading part of the loop through the trailing part of the loop (104);
iv) applying tension to the suture to force the leading part into engagement with the trailing part whilst tissue is sutured (106);
v) and applying a suture clip to the leading part such that a peripheral surface engages or abuts the trailing part or surrounding tissue (108).

It will be appreciated that many modifications may be made to the described arrangements without departing from the invention as defined in the appended claims.

The suture material may be any suitable material, for example Nylon, cotton, catgut, steel, aluminium or others.

Needle (10) as shown in the Figures is curved which is preferred. However, a straight needle may be used.

The needle, loop of suture material and suture clip can be any size - ranging from several millimetres to small, semi-microscopic systems.

The clip can be of any squeezable, lockable or crushable material. The alternative clip may be provided with means enabling it to be adhesively fixed to the suture material. The clip may even be welded to suture material.

The material of the clip may be dissolvable or non-dissolvable.

It will be appreciated that variations to the described method may also be made, for example a running lock suture may be formed with the suture material passing several times through the tissue to be sutured. The clip would then be applied to the last of the running lock sutures.

Other variations to the described arrangements will be seen by the skilled reader.

## Claims

1. Apparatus for suturing, comprising a closed loop (12) of suture material with leading and trailing parts (18,20), and a suture clip (24), the suture clip comprising a body (26) having a recess or opening (29,34) for receiving two sections (30A,30B) of the loop of suture material, means (31A,31B) for engaging two sections of the loop of suture material when received in the recess or opening, **characterised in that** the suture clip body further has a peripheral surface (32) for engaging or abutting the trailing part (20) of the loop or surrounding tissue to lock the two sections of the suture loop relative to the trailing part (20) after a suture has been formed, and **in that** the peripheral surface has a profile for preventing the trailing part from riding over the suture clip.

2. Apparatus for suturing according to claim 1, in which the body has a tubular portion with a bore extending therethrough providing said opening.

3. Apparatus for suturing according to claim 1, in which the body has a grooved portion into which two loop sections are receivable.

4. Apparatus according to any one of claims 1 to 3, in which the engaging means comprises a pair of opposed surfaces of the body, the opposed surfaces being movable between an inoperative position where suture loop sections are not engaged and an operative position where suture loop sections are engaged.

5. Apparatus according to claim 4, in which the opposed surfaces move from the inoperative to operative positions through plastic deformation within the body.

6. Apparatus according to claim 5 when appendent to claim 2, in which the tubular portion of the body is crushable.

7. Apparatus according to claim 5 when appendent to claim 3, in which sides of the grooved portion are foldable to close the groove opening.

8. Apparatus according to claim 4, in which the opposed surfaces move from the inoperative to operative positions through elastic deformation or hinged movement within the body.

9. Apparatus according to claim 8, further comprising a locking member or means for maintaining the opposed surfaces in the operative position.

10. Apparatus according to claim 9, in which the locking means comprises adhesive applied to one or other opposed surface for adhering to suture loop sections.

11. Apparatus according to any one of the preceding claims, in which the suture clip is bonded to the loop of suture material.

12. Apparatus according to claim 1, in which the profile provides an enlarged suture clip footprint in a plane substantially perpendicular to the alignment of suture loop sections when retained in the suture clip.

13. Apparatus according to claim 1 or 12, in which the profile comprises extensions which extend laterally of the rest of the body.

14. Apparatus according to claim 1,12 or 13 in which the profile is provided by a flared member which surrounds a region of contact between the leading and trailing parts of the loop.

15. Apparatus according to claim 14, in which the flared member is configured to bear against tissue surrounding the trailing part.

16. Apparatus according to any preceding claim, further comprising an applicator for the suture clip, the applicator having means for applying tension to the loop of suture material during suturing, and means for applying the suture clip and actuating the engaging means to secure the suture.

## Patentansprüche

1. Vorrichtung für das Legen einer Naht, welche eine geschlossene Schlinge (12) Nahtmaterial mit Führungs- und Nachlaufteilen (18, 20) und eine Nahtklemme (24) aufweist, wobei die Nahtklemme einen Körper (26) mit einer Vertiefung oder Öffnung (29, 34) für die Aufnahme von zwei Abschnitten (30A, 30B) der Nahtmaterial-Schlinge, eine Vorrichtung (31A, 31B) für die Ineingriffnahme von zwei Abschnitten der Nahtmaterial-Schlinge bei deren Aufnahme in der Vertiefung oder Öffnung aufweist, **dadurch gekennzeichnet, dass** der Nahtklemmen-Körper des Weiteren eine periphere Oberfläche (32) für die Ineingriffnahme oder das Anliegen des Nachlaufteils (20) der Schlinge oder des umgebenden Gewebes aufweist, um die zwei Abschnitte der Nahtschlinge relativ zum Nachlaufteil (20) fest zu verbinden, nachdem eine Naht gelegt worden ist, und dass die periphere Oberfläche ein Profil aufweist, um zu verhindern, dass der Nachlaufteil über die Nahtklemme hinaus läuft.

2. Vorrichtung für das Legen einer Naht nach Anspruch 1, bei welcher der Körper einen röhrenförmigen Abschnitt mit einer sich durch diesen hindurch erstreckenden Ausbohrung aufweist, welche die Öffnung ausbildet.

3. Vorrichtung für das Legen einer Naht nach Anspruch 1, bei welcher der Körper einen gerillten Abschnitt aufweist, in welchen zwei Schlingenabschnitte aufnehmbar sind.

4. Vorrichtung nach einem der Ansprüche 1 bis 3, bei welcher die Ineingriffnahme-Vorrichtung ein Paar gegenüber liegender Oberflächen des Körpers aufweist und die gegenüber liegenden Oberflächen zwischen einer nicht operativen Position, bei der Nahtschlingen-Abschnitte nicht in Eingriff genommen werden, und einer operativen Position, bei der Nahtschlingen-Abschnitte in Eingriff genommen werden, bewegbar sind.

5. Vorrichtung nach Anspruch 4, bei welcher sich die gegenüber liegenden Oberflächen durch eine plastische Verformung innerhalb des Körpers aus der nicht operativen in die operative Position bewegen.

6. Vorrichtung nach Anspruch 5, wenn zugehörig zu Anspruch 2, bei welcher der röhrenförmige Abschnitt des Körpers zusammendrückbar ist.

7. Vorrichtung nach Anspruch 5, wenn zugehörig zu Anspruch 3, bei welcher Seiten des gerillten Abschnitts faltbar sind, um die Rillenöffnung zu verschließen.

8. Vorrichtung nach Anspruch 4, bei welcher sich die gegenüber liegenden Oberflächen durch eine elastische Verformung oder Schwenkbewegung innerhalb des Körpers aus der nicht operativen in die operative Position bewegen.

9. Vorrichtung nach Anspruch 8, welche des Weiteren ein Verschlusselement oder eine Verschlussvorrichtung für das Halten der gegenüber liegenden Oberflächen in der operativen Position aufweist.

10. Vorrichtung nach Anspruch 9, bei welcher die Verschlussvorrichtung ein Klebemittel aufweist, das auf die eine oder die andere gegenüber liegende Oberfläche für das Anhaften an Nahtschlingen-Abschnitten aufgebracht ist.

11. Vorrichtung nach einem der vorhergehenden Ansprüche, bei welcher die Nahtklemme an der Schlinge des Nahtmaterials angebracht ist.

12. Vorrichtung nach Anspruch 1, bei welcher das Profil einen vergrößerten Nahtklemmen-Abdruck auf einer Ebene schafft, die im Wesentlichen senkrecht zur Ausrichtung von in der Nahtklemme gehaltenen Nahtschlingen-Abschnitten ist.

13. Vorrichtung nach Anspruch 1 oder 12, bei welcher das Profil Verlängerungen aufweist, die sich seitlich aus dem übrigen Körper erstrecken.

14. Vorrichtung nach Anspruch 1, 12 oder 13, bei welcher das Profil durch ein konisch erweitertes Element bereitgestellt wird, welches einen Kontaktbereich zwischen den Führungs- und Nachlaufteilen der Schlinge umgibt.

15. Vorrichtung nach Anspruch 14, bei welcher das konisch erweiterte Element so ausgelegt ist, dass es an Gewebe anliegt, welches den Nachlaufteil umgibt.

16. Vorrichtung nach einem der vorhergehenden Ansprüche, welche des Weiteren einen Applikator für die Nahtklemme umfasst, wobei der Applikator eine Vorrichtung für die Ausübung von Spannung auf die Schlinge des Nahtmaterials während des Legens der Naht und eine Vorrichtung für das Anbringen der Nahtklemme und die Betätigung der Ineingriffnahme-Vorrichtung für das sichere Anbringen der Naht aufweist.

## Revendications

1. Dispositif de suture, comprenant une boucle fermée (12) de matériau de suture avec des parties avant et arrière (18, 20), et une agrafe (24), l'agrafe comprenant un corps (26) ayant un évidement ou une ouverture (29, 34) pour recevoir deux sections (30A, 30B) de la boucle de matériau de suture, des moyens (31A, 31B) pour venir en prise avec deux sections de la boucle de matériau de suture lorsqu'elles sont reçues dans l'évidement ou l'ouverture, **caractérisé en ce que** le corps de l'agrafe a en outre une surface périphérique (32) pour venir en prise avec ou buter contre la partie arrière (20) de la boucle ou du tissu voisin pour bloquer les deux sections de la boucle de suture par rapport à la partie arrière (20) après qu'une suture a été formée, et **en ce que** la surface périphérique a un profil pour empêcher la partie arrière de passer sur l'agrafe.

2. Dispositif de suture selon la revendication 1, dans lequel le corps a une portion tubulaire avec un alésage qui s'étend à travers celle-ci fournissant ladite ouverture.

3. Dispositif de suture selon la revendication 1, dans lequel le corps a une section rainurée dans laquelle peuvent être reçues deux sections de boucle.

4. Dispositif selon l'une quelconque des revendications 1 à 3, dans lequel les moyens de mise en prise comprennent une paire de surfaces opposées du corps, les surfaces opposées étant mobiles entre une position au repos dans laquelle les sections de boucle de suture ne sont pas mises en prise et une position de fonctionnement dans laquelle les sections de boucle de suture sont mises en prise.

5. Dispositif selon la revendication 4, dans lequel les surfaces opposées se déplacent de la position au repos à la position de fonctionnement par déformation plastique à l'intérieur du corps.

6. Dispositif selon la revendication 5, lorsqu'elle dépend de la revendication 2, dans lequel la portion tubulaire du corps est compressible.

7. Dispositif selon la revendication 5, lorsqu'elle dépend de la revendication 3, dans lequel les côtés de la portion rainurée sont pliables pour fermer l'ouverture à rainures.

8. Dispositif selon la revendication 4, dans lequel les surfaces opposées se déplacent de la position au repos à la position de fonctionnement par déformation élastique ou par mouvement articulé à l'intérieur du corps.

9. Dispositif selon la revendication 8, comprenant en outre un élément de blocage ou des moyens pour maintenir les surfaces opposées dans la position de fonctionnement.

10. Dispositif selon la revendication 9, dans lequel les moyens de blocage comprennent un adhésif appliqué sur l'une ou l'autre des surfaces opposées pour adhérer aux sections de boucle de suture.

11. Dispositif selon l'une quelconque des revendications précédentes, dans lequel l'agrafe est fixée à la boucle de matériau de suture.

12. Dispositif selon la revendication 1, dans lequel le profil fournit une empreinte d'agrafe agrandie dans un plan sensiblement perpendiculaire à l'alignement des sections de boucle de suture lorsqu'elles sont retenues dans l'agrafe.

13. Dispositif selon la revendication 1 ou 12, dans lequel le profil comprend des extensions qui s'étendent latéralement par rapport au reste du corps.

14. Dispositif selon la revendication 1, 12 ou 13, dans lequel le profil est fourni par un élément évasé qui entoure une région de contact entre les parties avant et arrière de la boucle.

15. Dispositif selon la revendication 14, dans lequel l'élément évasé est configuré pour s'appuyer contre le tissu avoisinant la partie arrière.

16. Dispositif selon l'une quelconque des revendications précédentes, comprenant en outre un applicateur pour l'agrafe, l'applicateur ayant des moyens pour appliquer une tension à la boucle de matériau de suture lors de la suture, et des moyens pour appliquer l'agrafe et actionner les moyens de mise en prise pour fixer la suture.
